# EUROPEAN PATENT SPECIFICATION

(11) **EP 3 185 248 B1**
(45) Date of publication and mention of the grant of the patent: **06.11.2019**
(21) Application number: 15202411.3
(22) Date of filing: 23.12.2015
(51) Int. Cl.: H01B 1/02, A61B 5/00, A61N 1/05

(54) **PROCESS FOR ELECTRICALLY CONTACTING A COATED LEAD WITH A PARTICLE**
VERFAHREN ZUR ELEKTRISCHEN KONTAKTIERUNG EINER BESCHICHTETEN LEITUNG MIT EINEM TEILCHEN
PROCÉDÉ DE MISE EN CONTACT ÉLECTRIQUE D'UN CONDUCTEUR ENROBÉ AVEC UNE PARTICULE

(43) Date of publication of application: 28.06.2017
(73) Proprietor: Heraeus Deutschland GmbH & Co. KG, 63450 Hanau (DE)
(72) Inventor: Trötzschel, Jens, 63549 Ronneburg (DE); Schibli, Stefan, 60326 Frankfurt (DE)
(74) Representative: Herzog IP Patentanwalts GmbH

(56) References cited:
- WO-A1-2014/189028
- US-A1- 2013 068 513
- US-A1- 2013 338 745

## Description

### Technical Field

In general, the present invention relates generally to a process for electrically contacting a coated lead. More specifically, the invention relates to a process, an electrically contacted lead and a medical device comprising an electrically contacted lead.

### Background

Making electrical contact with an electrically conductive lead can often represent a significant challenge. In order to take advantage of beneficial properties of an electrical lead, such as good electrical conductivity and good durability, the contact to the lead itself must also display similar beneficial properties. Such considerations are particularly pertinent for example when electrically contacting leads in medical devices. In devices which are introduced into the body, it is desirable to employ thin leads, which can represent a challenge to electrically contact due their size. Furthermore, a very high value is placed on reliability in medical devices such as Cardiac Pacemakers, Implantable Cardioverter Defibrillation Devices and Cardiac Resynchronisation Devices, especially in terms of resistance to physical fatigue. Invasive surgery is required to implant medical devices into the body or remove or replace parts, and it is highly desirable for the individual components of the device to have a long working life in order to reduce the requirement for surgical intervention. Furthermore, it is desirable for the working life to have a low variance. One component of a medical device which is exposed to a particularly high amount of stress during normal operation is the lead and the electrical connections thereto.

Document US 7,364,479 B1 discloses a method for contacting a lead by crimping. Direct crimping can result in a contact which is lost over time due to physical movement. Furthermore, the direct crimping method is not suitable for multi-core leads since contact would be made to the cores indiscriminately.

Document US 2013/0338745 A1 discloses a method for contacting individual conductive cores of a cable which comprises multiple conductive cores. The individually coated conductive cores are fed through the lumens of a ring, and electrical contact is made with the individual cores by piercing. This method suffers at least from the disadvantages associated with moveable parts.

US patent application US 20131068513 A1 discloses the use of a metal/resin paste for making contacts in a multilayered wiring board.

US patent application US 2016/086687 A1 discloses conductive material having a high reaction rate and flux effect for use in a printed circuit board.

### Summary of the invention

The invention is generally based on the object of overcoming at least one of the problems encountered in the state of the art in relation to electrical contacts with leads.

More specifically, the invention is based on the object of providing a process for electrically contacting a lead, in particular which provides for one or more selected from the following group of advantages: an improved electrical contact, an improved durability and a more flexible process.

One object of the invention is to provide a process which is suitable for electrically contacting a thin lead, preferably whilst providing for one or more selected from the following group of advantages: an improved electrical contact, an improved durability and a more flexible process.

Another object of the invention is to provide a process which is suitable for electrically contacting a lead for use in a medical device, in particular a thin lead. In particular, it is preferred for electrically contacting a lead in a bio-compatible device, preferably whilst providing for one or more selected from the following group of advantages: an improved electrical contact, an improved durability and a more flexible process. One aspect of this object is to provide a process for connecting a lead which fulfils the requirements laid out in the "Guidance for the Submission of Research and Marketing Applications for Permanent Pacemaker Leads and for Pacemaker Lead Adaptor 510(k) Submissions" issued by the "Center for Devices and Radiological Health" of the US Food and Drug Administration. A further object is provide a method for electrically contacting a multi-core lead, in particular for discriminately contacting a single core thereof. This has long been attempted and there are various approaches provided in the art. Some of these have been discussed above. There remains, however, a need for further improvement of such electrical contacts and methods for making them.

### Detailed description of the invention

A contribution to achieving at least one of the above described objects is made by the subject matter of the category forming claims of the invention. A further contribution is made by the subject matter of the dependent claims of the invention which represent specific embodiments of the invention.

A contribution to achieving at least one of the above described objects is made by the following embodiments.
|1| Process according to the invention for electrically contacting a coated lead comprising the following steps:
   a. Providing a coated lead comprising an electrically conductive core and an electrically insulating coating;
   b. Providing a via hole in the electrically insulating coating in order to expose a section of the electrically conductive core;
   c. Introducing a first electrically conductive material into the via hole such that it contacts at least a part of the exposed section of the electrically conductive core;
   d. Applying a further electrically conductive material to the coated lead such that it contacts at least a part of the first conductive material.
|2| The process according to the invention according to embodiment |1|, wherein the first electrically conductive material comprises an electrically conductive particle, preferably a micro-particle. In one aspect of this embodiment, the first electrically conductive material is one or more electrically conductive particles, preferably is one conductive particle. In one aspect of this embodiment, the first electrically conductive material comprises 1 to 20 electrically conductive particles, preferably 1 to 5 conductive particles, more preferably 1 or 2 conductive particles, most preferably one conductive particle.
|3| The process according to the invention according to embodiment |2|, wherein the particle has a diameter in the range from about 5 to about 500 µm, preferably in the range from about 10 to about 200 µm, more preferably in the range from about 20 to about 100 µm, most preferably in the range from about 30 to about 70 µm.
|4| The process according to this disclosure according to embodiment |2| or |3|, wherein the particle has a diameter which is greater than the thickness of the electrically insulating coating, preferably a diameter in the range from more than about 1 to about 4 times, more preferably in the range from about 1.2 to about 3 times, further more preferably from about 1.3 to about 2.7 times, most preferably from about 1.4 to about 2.3 times the thickness of the coating.
|5| The process according to this disclosure according to any of the embodiments |2| to |4|, wherein the particle is spherical.
|6| The process according to the invention according to any of the embodiments |2| to |5|, wherein the introduction of the particle comprises sprinkling, dipping, dusting, the use of tweezers, or a combination of two or more selected therefrom, wherein dipping is prefered.
|7| The process according to this disclosure according to any of the preceding embodiments, wherein the first electrically conductive material has a mass in the range from about 0.01 to about 20 µg, preferably in the range from about 0.05 to about 10 µg, more preferably in the range from about 0.08 to about 8 µg, most preferably in the range from about 0.1 to about 5 µg.
|8| The process according to the invention according to any of the preceding embodiments, wherein the first electrically conductive material comprises a metal.
|9| The process according to this disclosure according to any of the preceding embodiments, wherein the first electrically conductive material is the same material as the electrically conductive core.
|10| The process according to the invention according to any of the preceding embodiments, wherein the second electrically conductive material is applied by solid deformation.
|11| The process according to this disclosure according to any of the preceding embodiments, wherein the application of the second electrically conductive material in step d. comprises one or more selected from the group consisting of: crimping, notching, bending, twisting, screwing and press fitting, preferably crimping.
|12| The process according to this disclosure according to any of the preceding embodiments, wherein the second electrically conductive material is a metal.
|13| The process according to the invention according to any of the preceding embodiments, wherein the lead has a diameter less than about 2 mm, preferably less than about 1.5 mm, more preferably less than about 1 mm, most preferably less than about 0.7 mm. In some cases, the thickness of the lead is as little as 0.1 mm or more.
|14| The process according to this disclosure according to any of the preceding embodiments, wherein the electrically conductive core is a metal.
|15| The process according to this disclosure according to any of the preceding embodiments, wherein the electrically insulating coating is a polymer.
|16| The process according to the invention according to any of the preceding embodiments, wherein the lead comprises 2 or more electrically conductive cores, preferably 3 or more, more preferably 4 or more.
|17| The process according to the invention according to embodiment |16|, wherein 2 or more of the electrically conductive cores are electrically insulated from each other by an insulated material, preferably each of the conductive cores is insulated from each of the other conductive cores.
|18| The process according to the invention according to any of the preceding embodiments, wherein the via hole exposes a single conductive core.
|19| The process according to this disclosure according to any of the preceding embodiments, wherein the section of the electrically conductive core exposed by the via hole has a surface area in the range from about 0.001 to about 0.1 mm², preferably in the range from about 0.005 to about 0.08 mm², more preferably in the range from about 0.008 to about 0.04 mm².
|20| The process according to the invention according to any of the preceding embodiments, wherein the provision of the via hole in step b. is performed with a laser.
|21| An electrically contacted lead according to the invention obtainable by a method according to any of the preceding embodiments.
|22| An electrically contacted lead according to the invention comprising the following:
   a. an electrically conductive core
   b. an electrically insulating coating which coats the electrically conductive core;
   c. a via hole in the electrically insulating coating which exposes a section of the electrically conductive core;
   d. a first electrically conductive material in the via hole which contacts at least a part of the exposed section of the electrically conductive core;
   e. a further electrically conductive material which contacts at least a part of the first conductive material.

The features introduced in relation to the above process embodiments apply mutatis mutandis as preferred aspects of this embodiment.
|23| A medical device according to the invention comprising a contacted lead according to embodiment |21| or |22|.
|24| A medical device according to this disclosure according to embodiment |23|, selected from the group consisting of: a pacemaker, a neuro-stimulator, a measuring device, and a defibrillator.
|25| A use of electrically conductive particles according to the invention for contacting a coated lead, wherein the electrically conductive particles satisfy one or more of the following criteria:
   a. D₅₀ in the range from about 10 to about 100 µm, preferably in the range from about 15 to about 60 µm, more preferably in the range from about 20 to about 50 µm;
   b. D₁₀ in the range from about 5 to about 40 µm, preferably in the range from about 10 to about 35 µm, more preferably in the range from about 15 to about 30 µm;
   c. D₉₀ in the range from about 40 to about 70 µm, preferably in the range from about 45 to about 65 µm, more preferably in the range from about 50 to about 60 µm;
   d. Difference D₉₀ - D₁₀ in the range from about 1 to about 25 µm, preferably in the range from about 3 to about 15 µm, more preferably in the range from about 5 to about 10 µm;
   e. A standard deviation of diameter in the range from about 0.5 to about 10 µm, preferably in the range from about 1 to about 8 µm, more preferably in the range from about 2 to about 5 µm.

The preferred aspects of this embodiments, the following combinations are satisfied: a, b, c, d, a+b, a+c, a+d, b+c, b+d, c+d, a+b+c, a+b+d, a+c+d, b+c+d, a+b+c+d, e, a+e, b+e, c+e, d+e, a+b+e, a+c+e, a+d+e, b+c+e, b+d+e, c+d+e, a+b+c+e, a+b+d+e, a+c+d+e, b+c+d+e or a+b+c+d+e. The preferred features of the first electrically conductive material are preferred for the electrically conductive particles.

### Process for electrically contacting a lead

A contribution to achieving at least one of the above mentioned objects is made by a process for electrically contacting a lead. The process according to the invention preferably comprises the following steps:
a. Providing a coated lead comprising an electrically conductive core and an electrically insulating coating;
b. Providing a via hole in the electrically insulating coating in order to expose a section of the electrically conductive core;
c. Introducing a first electrically conductive material into the via hole such that it contacts at least a part of the exposed section of the electrically conductive core;
d. Applying a further electrically conductive material to the coated lead such that it contacts at least a part of the first conductive material.

In one embodiment, the lead is electrically contacted one by the process according to the invention. In another embodiment, the lead is electrically contacted two or more times by the process according to the invention.

The lead of step a. is the lead to be contacted. The conductive core is preferably longitudinal, extending along the lead. The electrically insulating coating preferably extends along the length of the lead, coating the electrically conductive core. The process provides points of electrical contact at two points along the lead such that in use the lead provides an electrical connection between the points of electrical contact.

The via hole of step b. is provided in the electrically insulating coating order to expose a section of the electrically conductive core. The via preferably does not extend to a significant extent into the electrically conductive core, preferably not more than about 10 µm, more preferably not more than about 1 µm, more preferably still not more than about 0.1 µm, most preferably the via hole does not extend into the electrically conductive core. The section of the electrically conductive core is preferably exposed in order to allow electrical connection thereto. The area of the electrically conductive core exposed is preferably sufficient to allow electrical contact.

The via hole may be provided mechanically or otherwise. Preferred methods of providing the via hole are cutting or laser ablation, preferably laser ablation.

The first electrically conductive material of step c. is introduced into the via hole in order to make contact with the electrically conductive core. Preferably, at least a part of the first electrically conductive material occupies at least a part of the volume of the via hole. The first electrically conductive material preferably spans the depth of the via hole in order to allow contact with the further electrically conductive material.

The first electrically conductive material is introduced by sprinkling, dipping, dusting, the use of tweezers, or a combination of two or more selected therefrom, wherein dipping is preferred. In the case of dipping, the coated lead is preferably dipped into electrically conductive particles satisfying on or more of the following:
a. D₅₀ in the range from about 10 to about 100 µm, preferably in the range from about 15 to about 60 µm, more preferably in the range from about 20 to about 50 µm;
b. D₁₀ in the range from about 5 to about 40 µm, preferably in the range from about 10 to about 35 µm, more preferably in the range from about 15 to about 30 µm;
c. D₉₀ in the range from about 40 to about 70 µm, preferably in the range from about 45 to about 65 µm, more preferably in the range from about 50 to about 60 µm;
d. Difference D₉₀ - D₁₀ in the range from about 1 to about 25 µm, preferably in the range from about 3 to about 15 µm, more preferably in the range from about 5 to about 10 µm.
e. A standard deviation of diameter in the range from about 0.5 to about 10 µm, preferably in the range from about 1 to about 8 µm, more preferably in the range from about 2 to about 5 µm.
In preferred aspects, the following combinations are satisfied: a, b, c, d, a+b, a+c, a+d, b+c, b+d, c+d, a+b+c, a+b+d, a+c+d, b+c+d, a+b+c+d, e, a+e, b+e, c+e, d+e, a+b+e, a+c+e, a+d+e, b+c+e, b+d+e, c+d+e, a+b+c+e, a+b+d+e, a+c+d+e, b+c+d+e or a+b+c+d+e. The preferred features of the first electrically conductive material are preferred for the electrically conductive particles.

The thickness of the first electrically conductive material is preferably the radial distance from the surface of the electrically conductive core to the surface of the first electrically conductive material radially most distant from the electrically conductive core. In one embodiment, the first electrically conductive material comprises a particle and the thickness is the diameter of the particle. It is preferred for the first electrically conductive material to be present in the via hole with a thickness which is not significantly less than the depth of the via hole. It is preferred for the thickness of the first electrically conductive material to be sufficient for contact to be made with the further electrically conductive material applied thereto, which is preferably applied mechanically thereto, either with no deformation or with slight deformation of the insulating coating. In one aspect of this embodiment, the thickness of the first electrically conductive material is 0.8 times or more, preferably 0.9 time or more, more preferably 1 time or more the thickness of the insulating coating. In another aspect of this embodiment, the thickness of the first electrically conductive material is greater than the thickness of the insulating coating, preferably at least more than 1, preferably at least about 1.3 times, more preferably at least about 1.5 times the thickness of the insulating coating.

The further electrically conductive material of the step d. is provided in contact with the first electrically conductive material in order to provide an electrical contact via the first electrically conductive material to the electrically conductive core. The further electrically conductive material preferably provides mechanical stability to the contact with the electrically conducting core.

It is preferred for the further electrically conductive material to be applied by solid deformation, preferably comprising one or more selected from the list consisting of: crimping, notching, bending, twisting, screwing and press fitting, preferably crimping.

### Coated lead

The coated lead preferably has a core/coating structure. The coated lead preferably has a longitudinal core extending along the lead and a coating which surrounds the core.

In one embodiment the electrically conductive core has a circular cross section. A circular cross section is preferably one which increases the ratio of cross sectional area to cross sectional circumference. In one embodiment the cross sectional area (CSA) divided by the square of the cross sectional circumference (CSC), expressed in the form (CSA)/(CSC)², is at least about 0.06, preferably at least about 0.07, more preferably at least about 0.075, most preferably at least about 0.077.

In one embodiment, the insulating coating has a thickness which is less than the radius of the electrically conductive core, preferably having a thickness which is in the range from about 0.01 to about 1 times, preferably in the range from about 0.1 to about 0.95 times, more preferably in the range from about 0.3 to about 0.9 times the radius of the electrically conductive core.

In one embodiment, the electrically conductive core preferably constitutes the major part of the coated lead, preferably by mass or by thickness or both.

In one embodiment, the cross sectional diameter of the lead is in the range from about 0.05 to about 2 mm, preferably in the range from about 0.1 to about 1 mm, more preferably in the range from about 0.2 to about 0.8 mm, most preferably in the range from about 0.3 to about 0.7 mm.

### Electrically conductive core

The electrically conductive core is responsible for electrical conductivity along the lead. The electrically conductive core is preferably of an electrically conductive material, preferably a metallic conductor. The electrically conductive core is preferably a metal. Preferred metals in this context are one or more selected form the group consisting of: an elemental metal and an alloy. Preferred metallic elements in this context are one or more selected from the group consisting of: Pt, Ir, Ta, Pd, Ti, Fe, Au, Mb, Nb, W, Ni and Ti. Preferred alloys in this context comprise one or more selected from the group consisting of: Pt, Ir, Ta, Pd, Ti, Fe, Au, Mb, Nb, W, Ni and Ti. Preferred alloys in this context are one or more selected from the group consisting of MP35, steel, and platinum/iridium alloy. The preferred steel is stainless steel, preferably a stainless steel suitable for biological applications, preferably 316L, 301 or 304, more preferably 316L. Preferred platimum/iridium alloys have a weight ratio of Pt:Ir in the range from about 60:40 to about 98:2, preferably in the range from about 70:30 to about 95:5, more preferably in the range from about 80:20 to about 90:10.

In one embodiment, the electrically conductive core has a diameter in the range from about 0.01 to about 3 mm, preferably in the range from about 0.013 to about 1.5 mm, more preferably in the range from about 0.02 to about 1 mm, most preferably in the range from about 0.05 to about 0.7 mm.

In one embodiment the electrically conductive core is formed of two or more electro-conductive strands, preferably 4 to 9 electrically conductive strands, wherein the strands are in electrical contact.

### Electrically insulating coating

The purpose of the electrically insulating coating is to provide an electrical insulation between the electrically conductive core and the surroundings. The insulating coating is preferably of an electrically insulating material, preferably a polymer. Preferred polymers are one or more selected from the group consisting of: a poly epoxide, a poly alkene, a polyester and a poly silicon. Preferred polymers are one or more selected from the group consisting of: epoxy resin, silicone, polyurethane, polystyrene, polyethylene, polyethylene terephthalate, polytetrafluoroethylene and ethylene tetra fluoro ethylene. Other polymers which provide an insulating layer may be employed as the insulating coating.

In one embodiment, the electrically insulating coating comprises two or more layers of insulating coating, preferably two or more layers of polymer. In one aspect of this embodiment, the insulating coating comprises two layers of different electrically insulating material.

The thickness of the electrically insulating coating is preferably in the range from about 3 to about 150 µm, more preferably in the range from about 5 to about 100 µm, further more preferably in the range from about 9 to about 70 µm, most preferably in the range from about 12 to about 40 µm.

### First electrically conductive material

The first electrically conductive material provided electrical contact between the electrically conductive core of the lead and the further electrically conductive material. The first electrically conductive material preferably exhibits metallic conductivity. The first electrically conductive material is preferably a metal. Preferred metals in this context are one or more selected form the group consisting of: an elemental metal and an alloy. Preferred metallic elements in this context are one or more selected from the group consisting of: Pt, Ir, Ta, Pd, Ti, Fe, Au, Mb, Nb, W, Ni and Ti. Preferred alloys in this context comprise one or more selected from the group consisting of: Pt, Ir, Ta, Pd, Ti, Fe, Au, Mb, Nb, W, Ni and Ti. Preferred alloys in this context are one or more selected from the group consisting of MP35, steel, and platinum/iridium alloy. The preferred steel is stainless steel, preferably a stainless steel suitable for biological applications, preferably 316L, 301 or 304, more preferably 316L. Preferred platimum/iridium alloys have a weight ratio of Pt:Ir in the range from about 60:40 to about 98:2, preferably in the range from about 70:30 to about 95:5, more preferably in the range from about 80:20 to about 90:10.

In one embodiment, the first electrically conductive material is the same material as the electrically conductive core.

In one embodiment, the first electrically conductive material is introduced into the via hole in the form of one or more particles, preferably a single particle. A particle preferably has a ratio of its larges Cartesian dimension to its lowest Cartesian dimension in the range from about 1 to about 3, preferably in the range from about 1 to about 2.5, more preferably in the range from about 1 to about 2, most preferably in the range from about 1 to about 1.5. The preferred shape of the particles is spherical or significantly spherical. Other shapes of the particles are also possible. In one aspect of this embodiment the particle(s) has/have a shape which increases the ratio of the volume of the particle to the surface area of the particle. It is preferred for the square of the volume of the particle (VOL) divided by the cube of the surface are of the particle (SA), expressed in the form (VOL)²/(SA)³ to be at least about 0.007, preferably at least about 0.008, more preferably at least about 0.0083, most preferably at least about 0.0085.

Preferred particles have a mass lower than about 50 µg, preferably lower than about 30 µg, more preferably less than about 20 µg, most preferably less than about 10 µg. Particles might have a mass as low as about 0.1 µg or more.

### Second electrically conductive material

The second electrically conductive material makes electrical contact with the first electrically conductive material and in this way is in electrical contact with the electrically conductive core. The second electrically conductive material preferably exhibits metallic conductivity. The second electrically conductive material is preferably a metal. Preferred metals in this context are one or more selected form the group consisting of: an elemental metal and an alloy. Preferred metallic elements in this context are one or more selected from the group consisting of: Pt, Ir, Ta, Pd, Ti, Fe, Au, Mb, Nb, W, Ni and Ti. Preferred alloys in this context comprise one or more selected from the group consisting of: Pt, Ir, Ta, Pd, Ti, Fe, Au, Mb, Nb, W, Ni and Ti. Preferred alloys in this context are one or more selected from the group consisting of MP35, steel, and platinum/iridium alloy. The preferred steel is stainless steel, preferably a stainless steel suitable for biological applications, preferably 316L, 301 or 304, more preferably 316L. Preferred platimum/iridium alloys have a weight ratio of Pt:Ir in the range from about 60:40 to about 98:2, preferably in the range from about 70:30 to about 95:5, more preferably in the range from about 80:20 to about 90:10.

In one embodiment, the second electrically conductive material is of the same material as the electrically conductive core or as the first electrically conductive material or both.

In one embodiment, the second electrically conductive material is a ring, preferably a crimpable ring.

In one embodiment, the second electrically conductive material has a mass in the range from about 0.05 mg to about 0.5 g, preferably in the range from about 0.1 mg to about 0.3 g, more preferably in the range from about 0.2 mg to about 1 mg.

### Multiple electrically conductive cores

In one embodiment, the lead comprises two or more electrically conductive cores, preferably with at least one of the electrically conductive cores, more preferably all of the electrically conductive cores being electrically insulated from the other electrically conductive core(s). The presence of more than one electrically conductive core in the lead preferably allows the lead to be used to create two or more isolated electrical circuits. In one embodiment, the lead comprises two or more electrically conductive cores, electrically insulated from each other, and two or more of the electrically conductive cores, preferably all of the electrically conductive cores, are electrically contacted by a process according to the invention. In one aspect of this embodiment, one or more of the electrically conductive cores, preferably all of the electrically conductive cores, is/are connected in two or more placed by a process according to the invention.

Where the lead comprises two or more electrically conductive cores, it is preferred for one or more, preferably all of the electrical contacts made by the process according to the invention to make contact with a single electrically conductive core.

Where more than one electrically conductive core is present, the preferred features of the electrically conductive core introduced above preferably apply to at least one of the electrically conductive cores, more preferably all of the electrically conductive cores.

In one embodiment, the coated lead comprises two or more coated wires with a core/coating structure, preferably twisted together to form a twisted lead. In one aspect of this embodiment, the ensemble of coated wires if further coated with an electrically insulating material.

### Electrically Conductive Particles

A contribution to achieving one or more of the above mentioned objects is made by a use of electrically conductive particles for contacting a lead. According to the invention, the electrically conductive particles should satisfy one or more of the following criteria:
a. D50 in the range from about 10 to about 100 µm, preferably in the range from about 15 to about 60 µm, more preferably in the range from about 20 to about 50 µm;
b. D₁₀ in the range from about 5 to about 40 µm, preferably in the range from about 10 to about 35 µm, more preferably in the range from about 15 to about 30 µm;
c. D90 in the range from about 40 to about 70 µm, preferably in the range from about 45 to about 65 µm, more preferably in the range from about 50 to about 60 µm;
d. Difference D90 - D10 in the range from about 1 to about 25 µm, preferably in the range from about 3 to about 15 µm, more preferably in the range from about 5 to about 10 µm;
e. A standard deviation of diameter in the range from about 0.5 to about 10 µm, preferably in the range from about 1 to about 8 µm, more preferably in the range from about 2 to about 5 µm.

The electrically conductive particles are preferably used as the first electrically conductive material in a process according to the invention. In one embodiment, a coated lead is dipped into a portion of the electro-conductive particles, preferably for introducing one or more particles into a via hole in the coating of the coated lead.

The electrically conductive particles are preferably obtained or obtainable by a spray process in which solid particles, preferably spherical or essentially spherical particles, are obtained through the solidification of molten material in a spray.

It may be necessary to obtain a portion of electrically conductive particles with a modified particle size distribution. One preferred method is sieving.

### Medical devices

A contribution to achieving one or more of the above mentioned objects is made by a medical device comprising a contacted lead according to the present invention. Preferred medical devices in this context are one or more selected form the group consisting of: a pacemaker, a defibrillator, a cardo resynchronisation device, a neuro-stimulator and a measuring device.

### Figures

The invention is now further illustrated using figures which are not to be considered as limiting the scope of the invention. In brief, the figures show the following:
Figures 1a to 1d show schematically the process for electrically contacting a coated lead
Figure 2 shows schematically a process according to the invention for contacting a coated lead.
Figure 3 shows schematically a pacemaker comprising a lead electrically connected according to the invention
Figure 4 shows schematically a lead with 3 electrically conductive cores, contacted six times according to the invention
Figure 5 shows a scanning electron microscope image of electrically conductive particles according to the invention

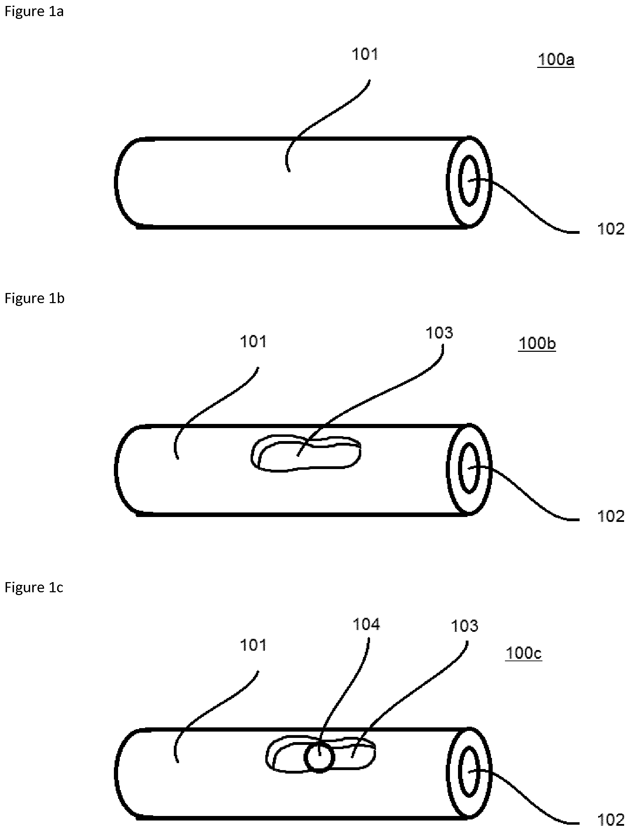

Figure 1a shows a coated lead 100a according to the invention. The coated lead 100a comprises an electrically conductive core 102 of electrically conductive material coated with an electrically insulating coating 101 of electrically insulating material. In this case, the electrically conductive material is a rhodium/platinum alloy (20 % rhodium and 80 % platinum, by mass) and the electrically conductive core has a diameter of 0.5 mm. In this case, the electrically insulating coating is 20 µm thick and the electrically insulating coating is of polyurethane.

Figure 1b shows 100b a coated lead with via hole 103. The coated lead with via hole 100b was obtained from the coated lead 100a by laser ablation. The via hole 103 extends through the electrically insulating coating 101 to expose a section of the surface of the electrically conductive core with a cross sectional area of 0.02 mm².

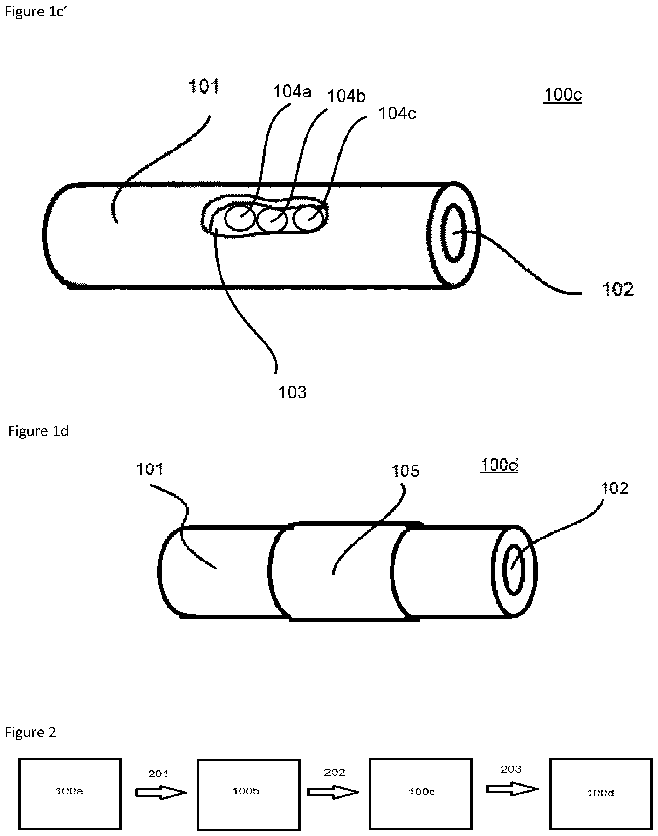

Figure 1c shows 100c a coated lead with a via hole 103 and a particle 104 in the via hole 103. The particle 104 is of an electrically conductive material, is spherical and has a diameter of 40 µm. The particle 104 is in electrical contact with the electrically conductive core 102. The particle 104 has been introduced into the via hole 103 using tweezers. In this case, the particle is of rhodium/platinum alloy (20 % rhodium and 30 % platinum, by mass).

Figure 1 c' shows 100c in which a total of three particles 104a, 104b & 104c have been introduced into the via hole.

Figure 1d shows 100d a coated lead with a via hole 103, a particle in the via hole and an electrically conductive ring 105 located over the via hole and particle. The conductive ring has been crimped around the lead so as to cover the via hole and the particle and to make electrical contact with the particle. In this case, the conductive ring is of rhodium/platinum alloy (20 % rhodium and 80 % platinum, by mass), and has a thickness of 0.5 mm.

Figure 2 shows schematically a process according to the invention for contacting a coated lead. Figure 2 shows how the items 100a, 100b, 100c & 100d are related by process steps. In step a), a coated lead 100a is provided. In step b) 201, a via hole is provided in the insulating coating of the coated lead 100a to obtain a coated lead with a via hole 100b. In step c) 202, a particle is introduced into the via hole to obtain a coated lead with a via hole and a particle in the via hole 100c. In step d) 203, an electrically conductive ring is crimped over the via hole and the particle to obtain the electrically contacted lead 100d.

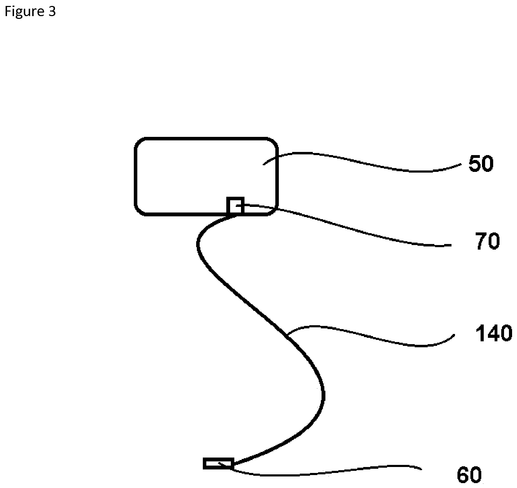

Figure 3 shows schematically a pacemaker 50 with a pulse generator 70, and a lead 140 comprising an electrode 60. The lead 140 connects the pulse generator 70 and the heart tissue via the electrode 60. The lead 140 has been electrically connected to the pulse generator 70 and the electrode 60 by a process according to the invention.

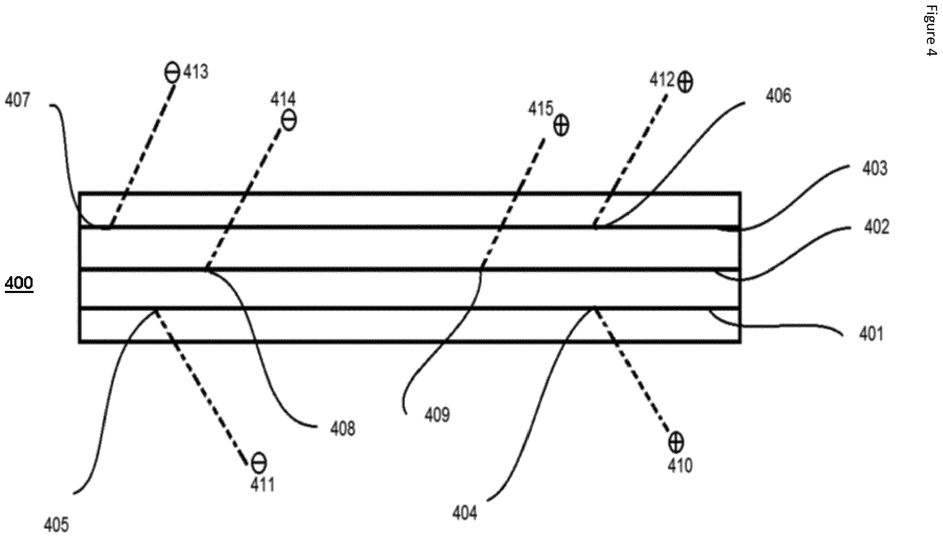

Figure 4 shows schematically a lead 400, comprising 3 electrically conductive cores 401, 402 & 403. Electrically conductive core 401 is electrically connected according to the invention at points 404 and 405 to allow an electrical circuit between notional terminals 410 and 411 respectively. Similarly electrically conductive core 402 is electrically connected according to the invention at points 408 and 409 to allow an electrical circuit between notional terminals 414 and 415 respectively. Similarly electrically conductive core 403 is electrically connected according to the invention at points 406 and 407 to allow an electrical circuit between notional terminals 412 and 413 respectively.

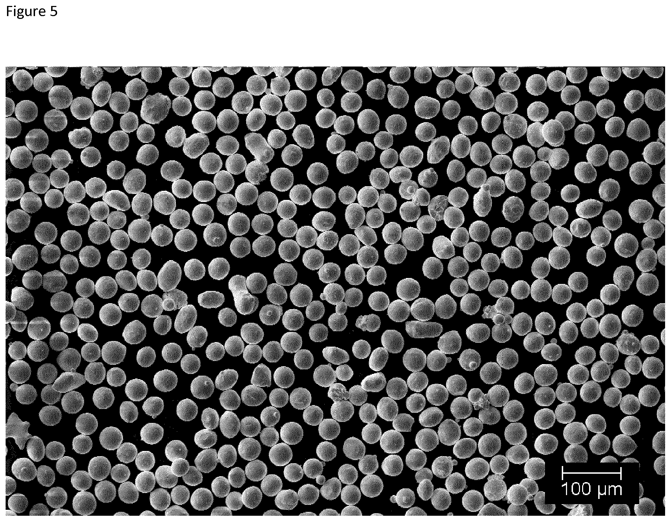

Figure 5 shows a scanning electron microscope image of a portion of electrically conductive particles according to the invention. The particles are of rhodium/platinum alloy (20 % rhodium, 80 % platinum, by mass) and have D₅₀=38 µm, D₁₀=35 µm, D₉₀=41 µm.

### Test Methods

### Surface area of particles

BET measurements to determine the specific surface area of electrically conductive particles are made in accordance with the static volumstric method according to DIN ISO 9277:2010. A Gemini 2390 (from Micromeritics) which works according to the SMART method (Sorption Method with Adaptive dosing Rate), is used for the measurement. As reference material Alpha Aluminium oxide CRM BAM-PM-102 available from BAM (Bundesanstalt für Material-forschung und -prüfung) is used. Filler rods are added to the reference and sample cuvettes in order to reduce the dead volume. The cuvettes are mounted on the BET apparatus. The saturation vapour pressure of nitrogen gas (N₂ 5.0) is determined. A Ig sample is weighed into a glass cuvette. The sample is kept at 100 °C for 2 hours in order to dry it. After cooling the weight of the sample is recorded. The glass cuvette containing the sample is mounted on the measuring apparatus. To degas the sample, it is evacuated at a pumping speed selected so that no material is sucked into the pump. The mass of the sample after degassing is used for the calculation. The dead volume is determined using Helium gas (He 4.6). The glass cuvettes are cooled to 77 K using a liquid nitrogen bath. For the adsorptive, N₂ 5.0 with a molecular cross-sectional area of 0.162 nm² at 77 K is used for the calculation. A multi-point analysis with 5 measuring points is performed and the resulting specific surface area given in m²/g.

### Fatigue Test

Fatigue resistance is measured according to the test described in "*prEN 45502 Parts 2 & 3 CEN*/*CENELEC, Active Implantable Medical Devices* - *Brady and Tachy Lead Tests Draft*/*Standard*".

### Examples

### Example 1 (inventive) - Contacting of a lead according to the invention

Electrical contacts were made to 15 cm long leads having a 0.2 mm diameter core of rhodium/platinum alloy (20 % rhodium, 80 % platinum, by mass) and a 20 µm coating of PTFE. 1 cm from each end of the lead, an electrical contact was made as follows: First, a via hole was made through the PTFE coating by laser ablation using a Varydisk laser available from Dausinger + Giesen GmbH. The via hole had a circular cross section of 60 µm diameter. Second, a portion of spherical rhodium/platinum alloy (20 % rhodium, 80 % platinum, by mass) particles with D₅₀=38 µm, D₁₀=35 µm, D₉₀=41 µm, commercially available from Heraeus Deutschland GmbH & Co. KG, was provided and the lead was dipped into the portion of particles in order to introduce a particle into the via hole. Third, a cylindrical ring of rhodium/platinum alloy (20 % rhodium, 80 % platinum, by mass) with outer diameter of 350 µm, an inner diameter of 260 µm and a cylinder length of 500 µm, available from Heraeus Deutschland GmbH & Co. KG, was threaded over the lead, positioned so as to cover the via hole with introduced particle and crimped tight onto the lead using a multi-jaw chuck.

### Example 2a (comparative) - Contacting of a lead by soldering

The lead was provided as in example 1 and electrical contact was made 1 cm from each end of each lead as follows: First, a via hole was made through the PTFE coating by laser ablation using a Varydisk laser available from Dausinger + Giesen GmbH. The via hole had a circular cross section of 60 µm diameter. Second, a cylindrical ring as in example 1 was threaded over the lead, positioned so as to cover the via hole and a contact was made between the ring and the core of the lead by soldering with gold at 700 °C.

### Example 2b (comparative) - Contacting of a lead by soldering

The lead was provided as in example 1 and electrical contact was made 1 cm from each end of each lead as follows: First, a via hole was made through the PTFE coating by laser ablation using a Varydisk laser available from Dausinger + Giesen GmbH. The via hole had a circular cross section of 60 µm diameter. Second, a cylindrical ring as in example 1, but with a 60 µm via hole was provided. The ring was threaded over the lead and positioned such that the via in the ring was over the via in the coating of the lead. Contact was made between the ring and the core of the lead by soldering through the two superimposed via holes with gold at 700 °C.

### Example 3 (comparative) - Contacting of a lead by crimping

A lead was provided as in example 1 and electrical contact was made 1 cm from each end of each lead as follows: First, a cylindrical ring as in example 1 was threaded over the lead and positioned 1 cm from the end of the lead. Second, the ring was crimped using a multi-jaw chuck to contact the ring with the core of the lead.

### Properties of the contacted leads

For each of the examples (1, 2a, 2b & 3), 5 leads were contacted as described. Each contacted lead was tested for fatigue resistance. The success rate of contacting the leads and the results for fatigue resistance are shown in table 1.

**Table 2**

| Example | Successful contacting ? | Fatigue resistance |
|---|---|---|
| 1 | Yes, in all 5 samples | ++ |
| 2a | 2 leads contacted, 3 failed | -- |
| 2b | 3 leads contacted, 2 failed | -- |
| 3 | 4 leads contacted, 1 failed | -- |

| | | |
|---|---|---|
| ++ Very good fatigue resistance (no failures after 400,000,000 cycles), -- very poor fatigue resistance (failed after an average of less than 300,000 cycles) | | |

### REFERENCE LIST

- 100a: coated lead
- 100b: coated lead with via hole
- 100c: coated lead with via hole and particle in the via hole
- 100d: contacted lead
- 101: electrically insulating coating
- 102: electrically conductive core
- 103: via hole
- 104: electrically conductive particle
- 105: electrically conductive ring
- 201: step b) - providing a via hole
- 202: step c) - introducing the first electrically conductive material into the via hole
- 203: step d) - applying the second electrically conductive material
- 50: pacemaker
- 70: pulse generator
- 140: lead
- 60: electrode

## Claims

1. A process for electrically contacting a coated lead comprising the following steps:
a. Providing a coated lead comprising an electrically conductive core and an electrically insulating coating;
b. Providing a via hole in the electrically insulating coating in order to expose a section of the electrically conductive core;
c. Introducing a first electrically conductive material into the via hole such that it contacts at least a part of the exposed section of the electrically conductive core;
d. Applying a further electrically conductive material to the coated lead such that it contacts at least a part of the first conductive material.

2. The process according to claim 1, wherein the first electrically conductive material comprises an electrically conductive particle.

3. The process according to claim 2, wherein the particle has a diameter in the range from about 5 to about 500 µm.

4. The process according to claim 2 or 3, wherein the introduction of the particle comprises sprinkling, dipping, dusting, the use of tweezers, or a combination of two or more selected therefrom.

5. The process according to any of the preceding claims, wherein the first electrically conductive material comprises a metal.

6. The process according to any of the preceding claims, wherein the second electrically conductive material is applied by solid deformation.

7. The process according to any of the preceding claims, wherein the lead has a diameter less than about 2 mm.

8. The process according to any of the preceding claims, wherein the lead comprises 2 or more electrically conductive cores.

9. The process according to claim 7, wherein 2 or more of the electrically conductive cores are electrically insulated from each other by an insulated material.

10. The process according to any of the preceding claims, wherein the via hole exposes a single conductive core.

11. The process according to any of the preceding claims, wherein the provision of the via hole in step b. is performed with a laser.

12. An electrically contacted lead obtainable by a method according to any of the preceding claims.

13. An electrically contacted lead comprising the following:
a. an electrically conductive core
b. an electrically insulating coating which coats the electrically conductive core;
c. a via hole in the electrically insulating coating which exposes a section of the electrically conductive core;
d. a first electrically conductive material in the via hole which contacts at least a part of the exposed section of the electrically conductive core;
e. a further electrically conductive material which contacts at least a part of the first conductive material.

14. A medical device comprising a contacted lead according to claim 12 or 13.

15. A use of electrically conductive particles for contacting a coated lead, wherein the electrically conductive particles satisfy one or more of the following criteria:
a. D₅₀ in the range from about 10 to about 100 µm;
b. D₁₀ in the range from about 5 to about 40 µm;
c. D₉₀ in the range from about 40 to about 70 µm;
d. Difference D₉₀ - D₁₀ in the range from about 1 to about 25 µm;
e. A standard deviation of diameter in the range from about 0.5 to about 10 µm.

## Patentansprüche

1. Ein Verfahren zum elektrischen Kontaktieren einer beschichteten Leitung, beinhaltend die folgenden Schritte:
a. Bereitstellen einer beschichteten Leitung, die einen elektrisch leitfähigen Kern und eine elektrisch isolierende Beschichtung beinhaltet;
b. Bereitstellen eines Durchgangslochs in der elektrisch isolierenden Beschichtung, um einen Abschnitt des elektrisch leitfähigen Kerns freizulegen;
c. Einbringen eines ersten elektrisch leitfähigen Materials in das Durchgangsloch, so dass es mindestens einen Teil des freigelegten Abschnitts des elektrisch leitfähigen Kerns kontaktiert;
d. Aufbringen eines weiteren elektrisch leitfähigen Materials auf die beschichtete Leitung, so dass sie mindestens einen Teil des ersten leitfähigen Materials kontaktiert.

2. Das Verfahren nach Anspruch 1, wobei das erste elektrisch leitfähige Material ein elektrisch leitfähiges Teilchen beinhaltet.

3. Das Verfahren nach Anspruch 2, wobei das Teilchen einen Durchmesser im Bereich von etwa 5 bis etwa 500 µm hat.

4. Das Verfahren nach Anspruch 2 oder 3, wobei das Einbringen des Teilchens ein Berieseln, ein Eintauchen, ein Bestäuben, die Verwendung einer Pinzette, oder eine Kombination von zwei oder mehr davon beinhaltet.

5. Das Verfahren nach einem der vorhergehenden Ansprüche, wobei das erste elektrisch leitfähige Material ein Metall beinhaltet.

6. Das Verfahren nach einem der vorhergehenden Ansprüche, wobei das zweite elektrisch leitfähige Material durch Verformen eines Festkörpers aufgebracht wird.

7. Das Verfahren nach einem der vorhergehenden Ansprüche, wobei die Leitung einen Durchmesser von weniger als etwa 2 mm hat.

8. Das Verfahren nach einem der vorhergehenden Ansprüche, wobei die Leitung 2 oder mehr elektrisch leitfähige Kerne beinhaltet.

9. Das Verfahren nach Anspruch 7, wobei 2 oder mehr der elektrisch leitfähigen Kerne durch ein isoliertes Material elektrisch voneinander isoliert sind.

10. Das Verfahren nach einem der vorhergehenden Ansprüche, wobei das Durchgangsloch einen einzelnen leitfähigen Kern freilegt.

11. Das Verfahren nach einem der vorhergehenden Ansprüche, wobei das Bereitstellen des Durchgangslochs in Schritt b. mit einem Laser durchgeführt wird.

12. Eine elektrisch kontaktierte Leitung, die durch ein Verfahren nach einem der vorhergehenden Ansprüche erhältlich ist.

13. Eine elektrisch kontaktierte Leitung, die das Folgende beinhaltet:
a. einen elektrisch leitfähigen Kern
b. eine elektrisch isolierende Beschichtung, die den elektrisch leitfähigen Kern beschichtet;
c. ein Durchgangsloch in der elektrisch isolierenden Beschichtung, das einen Abschnitt des elektrisch leitfähigen Kerns freilegt;
d. ein erstes elektrisch leitfähiges Material in dem Durchgangsloch, das mindestens einen Teil des freigelegten Abschnitts des elektrisch leitfähigen Kerns kontaktiert;
e. ein weiteres elektrisch leitfähiges Material, das mindestens einen Teil des ersten leitfähigen Materials kontaktiert.

14. Ein medizinisches Gerät, beinhaltend eine kontaktierte Leitung nach Anspruch 12 oder 13.

15. Eine Verwendung von elektrisch leitfähigen Teilchen zum Kontaktieren einer beschichteten Leitung, wobei die elektrisch leitfähigen Teilchen eines oder mehrere der folgenden Kriterien erfüllen:
a. D₅₀ im Bereich von etwa 10 bis etwa 100 µm;
b. D₁₀ im Bereich von etwa 5 bis etwa 40 µm;
c. D₉₀ im Bereich von etwa 40 bis etwa 70 µm;
d. Differenz D₉₀ - D₁₀ im Bereich von etwa 1 bis etwa 25 µm;
e. Eine Standardabweichung eines Durchmessers im Bereich von etwa 0,5 bis etwa 10 µm.

## Revendications

1. Procédé de mise en contact électrique d'un conducteur revêtu comprenant les étapes suivantes :
a. Fourniture d'un conducteur revêtu comprenant un noyau électriquement conducteur et un revêtement électriquement isolant ;
b. Prévoir un trou de passage dans le revêtement électriquement isolant afin d'exposer une section du noyau électriquement conducteur ;
c. Introduire un premier matériau électriquement conducteur dans le trou de passage de telle sorte qu'il entre en contact avec au moins une partie de la section exposée du noyau électriquement conducteur ;
d. Application d'un autre matériau électriquement conducteur sur le conducteur revêtu de telle sorte qu'il soit en contact avec au moins une partie du premier matériau conducteur.

2. Procédé selon la revendication 1, dans lequel le premier matériau électriquement conducteur comprend une particule électriquement conductrice.

3. Procédé selon la revendication 2, dans lequel la particule a un diamètre compris entre environ 5 et environ 500 µm.

4. Procédé selon la revendication 2 ou 3, dans lequel l'introduction de la particule comprend l'aspersion, le trempage, le saupoudrage, l'utilisation d'une pince à épiler, ou une combinaison de deux ou plusieurs de ces éléments choisis parmi ceux-ci.

5. Procédé selon l'une quelconque des revendications précédentes, dans lequel le premier matériau électriquement conducteur comprend un métal.

6. Procédé selon l'une quelconque des revendications précédentes, dans lequel le second matériau électriquement conducteur est appliqué par déformation solide.

7. Procédé selon l'une quelconque des revendications précédentes, dans lequel le plomb a un diamètre inférieur à environ 2 mm.

8. Procédé selon l'une quelconque des revendications précédentes, dans lequel le plomb comprend 2 ou plusieurs noyaux électriquement conducteurs.

9. Procédé selon la revendication 7, dans lequel 2 ou plusieurs des noyaux conducteurs de l'électricité sont isolés électriquement les uns des autres par un matériau isolé.

10. Procédé selon l'une quelconque des revendications précédentes, dans lequel le trou de passage expose un seul noyau conducteur.

11. Procédé selon l'une quelconque des revendications précédentes, dans lequel la réalisation du trou de passage à l'étape b. est réalisée avec un laser.

12. Conducteur en contact électrique pouvant être obtenu par un procédé selon l'une quelconque des revendications précédentes.

13. Conducteur à contact électrique comprenant les éléments suivants :
a. un noyau électriquement conducteur ;
b. un revêtement électriquement isolant qui recouvre le noyau électriquement conducteur ;
c. un trou de passage dans le revêtement électriquement isolant qui expose une partie du noyau électriquement conducteur ;
d. un premier matériau électriquement conducteur dans le trou de passage qui est en contact avec au moins une partie de la section exposée du noyau électriquement conducteur ;
e. un autre matériau électriquement conducteur qui est en contact avec au moins une partie du premier matériau conducteur.

14. Dispositif médical comprenant une sonde de contact selon la revendication 12 ou 13.

15. Utilisation de particules électriquement conductrices pour mettre en contact un conducteur revêtu, dans laquelle les particules électriquement conductrices satisfont un ou plusieurs des critères suivants :
a. Dso dans la plage d'environ 10 à environ 100 µm ;
b. D₁₀ dans la plage d'environ 5 à environ 40 µm;
c. D₉₀ dans la gamme d'environ 40 à environ 70 µm;
d. Différence D₉₀ - D₁₀ dans la plage d'environ 1 à environ 25 µm ;
e. Écart-type du diamètre compris entre environ 0,5 et environ 10 µm.
